(19) Europäisches Patentamt
European Patent Office
Office européen des brevets

(11) Numéro de publication : **0 005 385**
**B1**

(12) # FASCICULE DE BREVET EUROPÉEN

(45) Date de publication du fascicule du brevet : **01.09.82**

(51) Int. Cl.³ : **C 07 D295/18**, A 61 K 31/495

(21) Numéro de dépôt : **79400214.7**

(22) Date de dépôt : **03.04.79**

(54) **Dérivés de la pipérazine méthanimine, leur procédé de préparation et leurs applications en thérapeutique.**

(30) Priorité : **27.04.78 GB 16744/78**

(43) Date de publication de la demande : **14.11.79 (Bulletin 79/23)**

(45) Mention de la délivrance du brevet : **01.09.82 Bulletin 82/35**

(84) Etats contractants désignés : **BE CH DE FR GB IT LU NL SE**

(56) Documents cités :
**BE A 726 123**
**HOUBEN-WEYL « Methoden der organischen Chemie » 4. édition, vol. XI/2 1958, GEORG THIEME VERLAG, Stuttgart**

(73) Titulaire : **LAROCHE NAVARRON S.A. Société dite: 20, Rue Jean Jaurès F-92800 Puteaux (FR)**

(72) Inventeur : **Pascal, Jean-Claude 11-15 Allée des Hautes Bruyères F-94240 Cachan (FR)**
Inventeur : **Pinhas, Henri 39 Rue Truffaut F-75000 Paris (FR)**

(74) Mandataire : **Polus, Camille et al c/o Cabinet Lavoix 2, Place d'Estienne d'Orves F-75441 Paris Cedex 09 (FR)**

**EP 0 005 385 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Dérivés de la pipérazine méthanimine, leur procédé de préparation et leurs applications en thérapeutique.

La présente invention concerne de nouveaux dérivés de la pipérazine méthanimine, leur procédé de préparation et leurs applications en thérapeutique.

Le brevet belge 726123 décrit des benzamidines présentant en position para un groupe alcoxy. Ces composés sont décrits comme ayant une activité anthelmintique.

La présente invention a pour objet des composés de formule générale

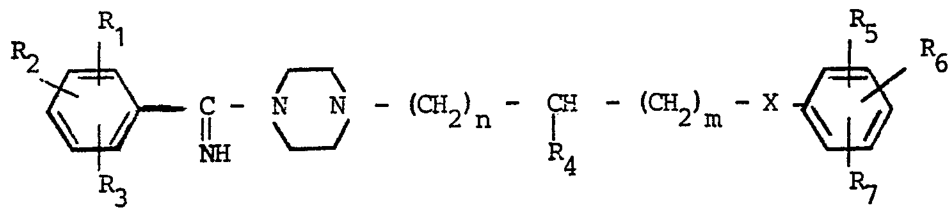

(I)

dans laquelle :

— les substituants $R_1$, $R_2$ et $R_3$ représentent indépendamment l'un de l'autre un atome d'hydrogène, un atome d'halogène, un radical trifluorométhyle, un radical alcoyle en $C_1$ à $C_6$ linéaire ou ramifié, un radical benzyloxy, ou un radical hydroxy.

— les substituants $R_5$, $R_6$ et $R_7$ représentent indépendamment l'un de l'autre un atome d'hydrogène, un atome d'halogène, ou un radical alcoyle en $C_1$ à $C_6$ linéaire ou ramifié.

— le substituant $R_4$ représente un atome d'hydrogène ou un radical alcoyle en $C_1$ à $C_6$.

— *m* est égal à 0 ou 1 et n est égal à 1 ou 2.

— X représente un atome d'oxygène, de soufre ou une simple liaison, ainsi que leurs sels d'addition avec des acides pharmaceutiquement acceptables.

Les sels d'addition peuvent être ceux formés par exemple avec les acides halohydrique, sulfurique, nitrique, phosphorique, formique, acétique, maléique, fumarique, méthane sulfonique, lactique, succinique, tartrique et des sels métalliques acides, tels que l'orthophosphate disodique et le sulfate monopotassique. Les bases libres peuvent exister sous forme hydratée ou pratiquement anhydre.

Une classe particulière de composés de formule I est celle constituée par les composés dans lesquels

— les substituants $R_1$, $R_2$, et $R_3$ représentent indépendamment l'un de l'autre un atome d'hydrogène, un atome d'halogène, un radical trifluorométhyle, un radical alcoyle en $C_1$ à $C_4$, un radical benzyloxy ou un radical hydroxy,

— les substituants $R_5$, $R_6$, et $R_7$ représentent indépendamment l'un de l'autre un atome d'hydrogène, un atome d'halogène ou un radical alcoyle en $C_1$ à $C_4$,

— le substituant $R_4$ représente un atome d'hydrogène ou un radical méthyle,

— *m* est égal à 0 ou 1 et n est égal à 1 ou 2,

— X représente un atome d'oxygène, de soufre ou une simple liaison, ainsi que leurs sels d'addition avec des acides pharmaceutiquement acceptables.

Les composés de formule I peuvent être préparés par réaction d'un iminoate d'alcoyle inférieur de formule

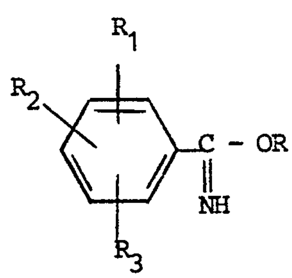

(II)

dans laquelle R est un radical alcoyle inférieur, notamment en $C_1$-$C_2$, et $R_1$, $R_2$, $R_3$ ont les significations données pour la formule I, sur une pipérazine de formule

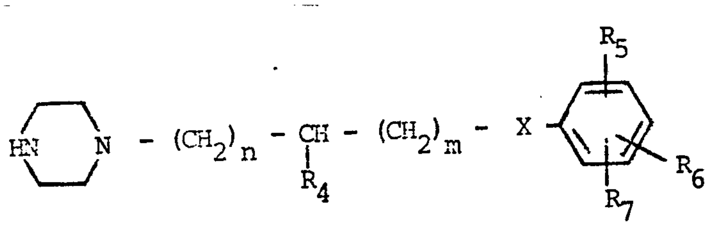

(III)

dans laquelle $R_4$, $R_5$, $R_6$, $R_7$, *m* et *n*, ont la signification donnée pour la formule I, en solution alcoolique à chaud.

Les composés de formule II s'obtiennent de manière classique par réaction d'un nitrile de formule

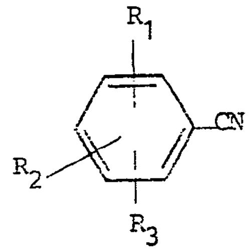

(IV)

en solution dans un mélange oxyde d'éthyle-alcool (notamment méthanol ou éthanol) en présence d'acide chlorhydrique.

Les composés de formule III peuvent être obtenus par réaction d'un dérivé halogéné de formule

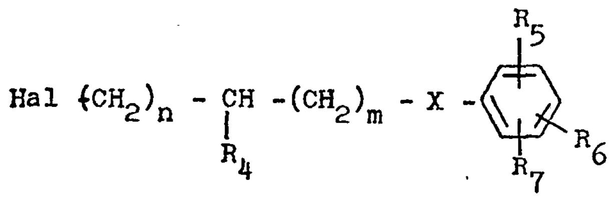

(V)

dans laquelle $R_4$, $R_5$, $R_6$, $R_7$, X, *m* et *n* ont les significations données pour la formule I, et Hal représente un halogène, avantageusement le chlore ou le brome, avec la pipérazine.

Cette réaction peut être effectuée dans un solvant tel que la diméthylacétone, l'éthylméthylcétone, l'eau, l'éthanol. On opère de préférence à la température de reflux du solvant en présence d'un accepteur d'acide, tel qu'un carbonate alcalin.

Quand X représente un oxygène ou un soufre, les composés de formule V s'obtiennent par réaction d'un phénol ou d'un thiophénol de formule

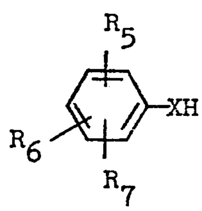

(VI)

dans lequel $R_5$, $R_6$, $R_7$ et X ont la signification donnée ci-dessus sur un dérivé dihalogéné de formule

$$Hal—(CH_2)_n—\underset{R_4}{\underset{|}{C}}H—(CH_2)_m—Hal \qquad (VII)$$

dans laquelle $R_4$, *m*, *n* et Hal ont la signification donnée précédemment, dans un solvant tel que l'eau, un alcool, un mélange hydroalcoolique en présence d'un agent alcalin, généralement la soude.

Les méthodes de préparation des dérivés de formule V dans laquelle X est une simple liaison sont couramment décrites dans la littérature.

En variante, les composés de formule I peuvent être aussi préparés :

— par réaction d'une benzamidine de formule

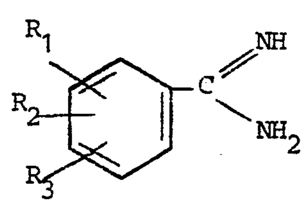

dans laquelle $R_1$, $R_2$ et $R_3$ ont les significations données pour la formule I, sur une pipérazine de

formule III en solution à reflux dans un solvant tel que le méthanol, éthanol, isopropanol,

— par réaction d'un iodhydrate de thiobenzimidate de S-méthyle de formule

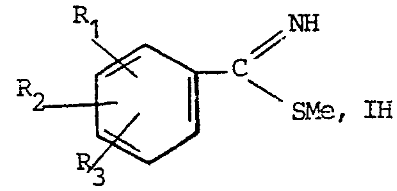

dans laquelle $R_1$, $R_2$ et $R_3$ ont les significations données pour la formule I, sur une pipérazine de formule III dans un solvant alcoolique. Les dérivés du thiobenzimidate de S-méthyle s'obtiennent d'une manière classique par réaction, dans l'acétone, d'un benzothioamide sur l'iodure de méthyle.

Les composés de formule I peuvent également être obtenus par réaction d'un organo magnésien de formule

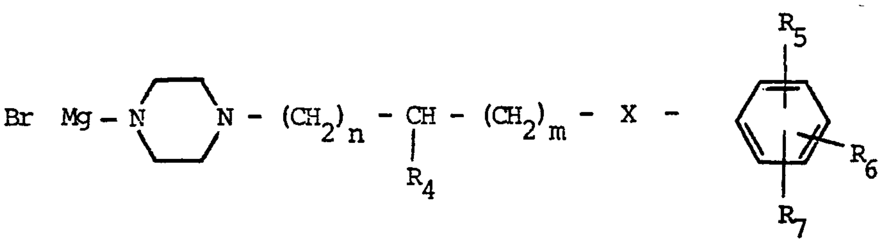

(VIII)

sur un nitrile de formule IV tel que défini ci-dessus et hydrolyse du produit réactionnel selon le schéma suivant :

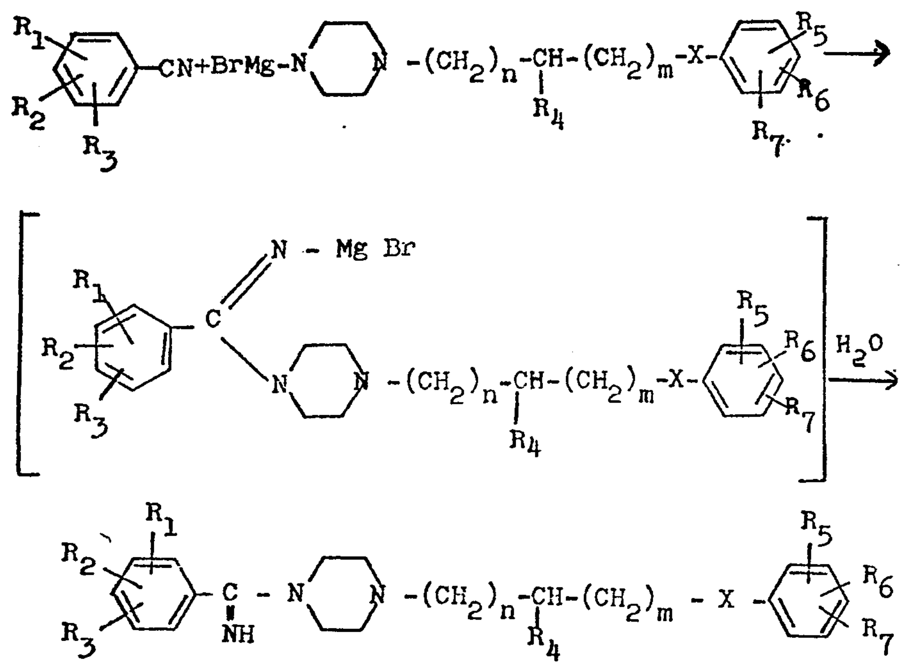


Les composés de formule VIII s'obtiennent de manière classique à partir des composés de formule III.

Les exemples suivants illustrent la présente invention.

Les exemples A à K illustrent la préparation des composés de formule III. Les exemples 1 à 20 illustrent la préparation des composés de formule I.

I. Préparation des composés de formule III

Exemple A

(Phényl-2 propyl)-1 pipérazine

On chauffe au reflux pendant 24 heures une solution de 344 g de pipérazine, 199 g de β-bromoisopropylbenzène dans 1 litre d'eau et 0,35 litre d'éthanol. Après refroidissement, l'amine formée est extraite trois fois avec 0,5 litre de chlorure de méthylène. Après lavage à l'eau et évaporation sous vide du solvant, on distille la phényl-2 propyl pipérazine qui bout à 163-165 °C sous 15 mm de Hg.

R.M.N. ($CCL_4$)
Ref : TMS
s(1H) 1,1 ppm ; m(6H) 2,3 ppm
d(3H) 1,2 ppm ; m(5H) 2,7 ppm
s(5H) 7,2 ppm

Exemple B

[(Diméthyl-2,6 phénoxy)-2 éthyl]-1 pipérazine

a) (diméthyl-2,6 phénoxy)-2 bromo-1 éthane

A une solution contenant 80 g de soude dans 1 litre d'eau et 0,5 litre d'éthanol, on ajoute 245 g de diméthyl-2,6 phénol en solution dans 0,5 litre d'éthanol, puis 750 g de dibromoéthane. On chauffe à reflux sous bonne agitation le mélange pendant 48 heures. On extrait au chlorure de méthylène. Après lavage avec une solution de soude diluée, puis d'eau, on évapore le solvant sous vide. Le (diméthyl-2,6 phénoxy)-2 bromo-1 éthane bout à 148-150 °C sous 25 mm de Hg.

b) [(diméthyl-2,6 phénoxy)-2 éthyl]-1 pipérazine

Dans 1 litre de méthyléthylcétone, on ajoute 132 g de (diméthyl-2,6 phénoxy)-2 bromo-1 éthane, 85 g d'iodure de sodium, 80 g de carbonate de potassium et 200 g de pipérazine. On chauffe sous agitation au reflux pendant 24 heures. Après refroidissement, le mélange est filtré, puis lavé trois fois avec 300 ml de benzène. Après évaporation du benzène, le dérivé de pipérazine est distillé. $E_{25} = 190$ °C.

En opérant comme décrit aux exemples A et B, on a préparé les composés suivants :

C-[(Diméthyl-2,6 phénoxy)-3 propyl]-1 pipérazine
$E_{13} = 195$ °C
D-(Phénoxy-2 éthyl)-1 pipérazine
$E_{13} = 190$ °C
E-(Phénoxy-3 propyl)-1 pipérazine
$E_{13} = 190$ °C
F-[(Méthyl-2 phénoxy)-2 éthyl]-1 pipérazine
$E_{0,2} = 114\text{-}116$ °C
G-(Phényl-2 éthyl)-1 pipérazine
$E_{13} = 160\text{-}165$ °C
H-(Phényl-3 propyl)-1 pipérazine
$E_{25} = 185\text{-}190$ °C
I-(Phénylthio-2 éthyl)-1 pipérazine
$E_{0,05} = 140\text{-}141$ °C
J-(Phénoxy-2 méthyl-1 éthyl)-1 pipérazine
$E_{0,1} = 104\text{-}105$ °C
K-(Phénoxy-3 hydroxy-2 propyl)-1 pipérazine
$E_{13} = 205\text{-}207$ °C

II. Préparation des composés de formule I

Exemple 1

Dichlorhydrate de [(diméthyl-2,6 phénoxy)-2 éthyl]-1 (trifluorométhyl-3 benzimidoyl)-4 pipérazine

5,4 g de chlorhydrate de trifluorométhyl-3 phényl iminoate d'éthyle et 5 g de [(diméthyl-2,6 phénoxy)-2 éthyl]-1 pipérazine dans 50 ml de méthanol sont portés au reflux pendant 24 heures. Après refroidissement, on acidifie le mélange réactionnel et on évapore le solvant. Le dichlorhydrate de [(diméthyl-2,6 phénoxy)-2 éthyl]-1 (trifluorométhyl-3 benzimidoyl)-4 pipérazine recristallise dans l'éthanol absolu sous forme de cristaux blancs qui fondent à 202-204 °C.

R.M.N. (DMSO $d_6$, Ref. : TMS)
s (6H) 2,35 ppm ; m (10H) 3,8 ppm
t (2H) 4,3 ppm ; s (3H) 7,15 ppm, m (4H) 8,15 ppm
s (1H) 10,2 ppm ; s (1H) 10,4 ppm, s (1H) 12,7 ppm

## Exemple 2

Dichlorhydrate de [(diméthyl-2,6 phénoxy)-2 éthyl]-1 (benzimidoyl)-4 pipérazine

### Méthode 1

A 1,2 g de magnésium dans 10 ml d'éther éthylique, on ajoute 7,5 ml de bromure d'éthyle. Après dissolution du magnésium, on ajoute 0,03 mole de [(diméthyl-2,6 phénoxy)-2 éthyl]-1 pipérazine dans 100 ml d'éther éthylique, puis on porte le mélange réactionnel au reflux pendant 30 mn. Après refroidissement, on ajoute 10,3 g de benzonitrile dans 100 ml d'éther et on chauffe au reflux pendant 3 heures. On ajoute au mélange 100 ml d'une solution aqueuse de chlorure d'ammonium. La phase éthérée est lavée à l'eau. Après précipitation à l'éther chlorhydrique, le composé formé est recristallisé dans le méthanol et fond à 231-232 °C.

R.M.N. (DMSO $d_6$)
s (6H) 2,35 ppm ; m (10H) 3,7 ppm
t (2H) 4,35 ppm ; s (3H) 7,15 ppm
s (5H) 7,8 ppm ;
s (1H) 10,2 ; 10,4 ; 12,7 ppm

### Méthode 2

0,1 mole de chlorhydrate de benzamidine et 0,1 mole de [(diméthyl-2,6 phénoxy)-3 éthyl]-1 pipérazine dans 300 ml d'isopropanol, sont portés au reflux sous barbotage d'azote. On maintient le reflux 72 heures. Après refroidissement, on acidifie le milieu réactionnel avec du méthanol chlorhydrique. Après concentration du solvant, le précipité est essoré et recristallisé dans de l'éthanol anhydre. On obtient ainsi un produit analogue à celui obtenu par la méthode 1.

### Méthode 3

0,1 mole d'iodhydrate de thiobenzimidate de S-méthyle est mise en solution dans 100 ml d'éthanol anhydre. On ajoute 0,1 mole de [(diméthyl-2,6 phénoxy)-2 éthyl]-1 pipérazine. On chauffe à reflux pendant 12 heures. On évapore l'alcool et au résidu on ajoute 100 ml de solution de soude N. On extrait à l'éther, on lave à l'eau. Après acidification de la phase éthérée, le précipité obtenu est essoré et cristallisé dans l'éthanol anhydre. On obtient un produit analogue à celui obtenu par les méthodes 1 et 2.

En opérant comme à l'exemple 1 ou à l'exemple 2, on a préparé les composés suivants.

## Exemple 3

Dichlorhydrate de (phényl-2 éthyl)-1 (chloro-4 benzimidoyl)-4 pipérazine. F = 244-245 °C.

R.M.N. ($D_2O$)
s (4H) 7,7 ppm ; m (12H) 3,5 ppm
s (5H) 7,3 ppm

## Exemple 4

Dichlorhydrate de (phénoxy-2 éthyl)-1 (chloro-4 benzimidoyl)-4 pipérazine. F = 217-218 °C.

R.M.N. (DMSO $d_6$, $D_2O$)
s (4H) 7,85 ppm ; m (12H) 3,8 ppm
m (5H) 7,3 ppm

## Exemple 5

Dichlorhydrate de (phénoxy-2 éthyl)-1 (trifluorométhyl-3 benzimidoyl)-4 pipérazine. F = 217-218 °C.

R.M.N. ($D_2O$)
m (4H) 8,15 ppm ; m (5H) 7,4 ppm
t (5H) 4,55 ppm ; t (2H) 4,4 ppm
m (10H) 3,90 ppm

## Exemple 6

Dichlorhydrate de (phényl-2 propyl)-1 (benzimidoyl)-4 pipérazine. F = 216-217 °C.

R.M.N. ($D_2O$)
d (3H) 1,5 ppm ; s (5H) 7,9 ppm
s (5H) 7,6 ppm ; t (2H) 4,25 ppm
m (9H) 3,8 ppm ;

Exemple 7

Dichlorhydrate de (phénoxy-3 propyl)-1 (dichloro-2,6 benzimidoyl)-4 pipérazine. F : 210-211 °C.

R.M.N. ($D_2O$)
m (2H) 2,4 ppm ; m (4H) 4,4 ppm
s (3H) 7,9 ppm ; m (8H) 3,8 ppm
m (5H) 7,4 ppm ;

Exemple 8

Dichlorhydrate de [(diméthyl-2,6 phénoxy)-2 éthyl]-1 (dichloro-2,6 benzimidoyl)-4 pipérazine. F = 250 °C.

R.M.N. ($D_2O$)
s (6H) 2,35 ppm ; t (2H) 4,3 ppm
m (10H) 3,85 ppm ; s (3H) 7,9 ppm
s (3H) 7,3 ppm ;

Exemple 9

Dichlorhydrate de (phénoxy-2 éthyl)-1 (dichloro-2,6 benzimidoyl)-4 pipérazine. F = 250 °C.

R.M.N. ($D_2O$)
s (3H) 7,8 ppm ; m (12H) 3,7 ppm
m (5H) 7,4 ppm ;

Exemple 10

Dichlorhydrate de [(diméthyl-2,6 phénoxy)-3 propyl]-1 dichloro-2,6 benzimidoyl)-4 pipérazine. F = 245 °C.

R.M.N. (DMSO $d_6$)
s (6H) 2,35 ppm ; s (3H) 7,15 ppm
s (1H) 11,6 ; 10,8 ; 11 ppm ; s (3H) 7,9 ppm
m (14H) 3,7 ppm

Exemple 11

Dichlorhydrate de [(méthyl-2 phénoxy)-2 éthyl]-1 (benzimidoyl)-4 pipérazine. F = 240-241 °C.

R.M.N. ($D_2O$)
s (3H) 2,35 ppm ; m (10H) 3,85 ppm
t (2H) 4,3 ppm ; s (5H) 7,9 ppm
m (4H) 7,3 ppm ;

Exemple 12

Dichlorhydrate de [(diméthyl-2,6 phénoxy)-2 éthyl]-1 (chloro-4 benzimidoyl)-4 pipérazine. F = 214-215 °C.

R.M.N. (DMSO $d_6$)
s (6H) 2,35 ppm ; m (10H) 3,85 ppm
t (2H) 4,3 ppm ; s (3H) 7,3 ppm
s (4H) 7,9 ppm ; s (1H) 10 ; 10,3 ; 12,5 ppm

Exemple 13

Dichlorhydrate de [(diméthyl-2,6 phénoxy)-3 propyl]-1 (trifluorométhyl-3 benzimidoyl)-4 pipérazine. F = 250 °C.

R.M.N. ($D_2O$)
s (6H) 2,35 ppm ; m (14H) 3,8 ppm

s (3H) 7,3 ppm ; m (4H) 8,2 ppm

Exemple 14

Dichlorhydrate de (phényl-3 propyl)-1 (trifluorométhyl-3 benzimidoyl)-4 pipérazine. F = 223-225 °C.

R.M.N. ($D_2O$)
s (5H) 7,6 ppm ; m (2H) 2,5 ppm
s (4H) 8,2 ppm ; m (12H) 3,8 ppm

Exemple 15

Dichlorhydrate de (phényl-3 propyl)-1 (benzimidoyl)-4 pipérazine. F = 245-247 °C.

R.M.N. (MeOH $d_4$)
s (5H) 7,75 ppm ; m (2H) 2,2 ppm
t (2H) 4,25 ppm ; s (5H) 7,3 ppm
t (2H) 2,7 ppm ; m (8H) 3,5 ppm

Exemple 16

Dichlorhydrate de [(diméthyl-2,6 phénoxy)-3 propyl]-1 benzimidoyl-4 pipérazine. F = 228-230 °C.

R.M.N. (DMSO $d_6$)
s (6H) 2,35 ppm ; s (5H) 7,9 ppm
s (3H) 7,3 ppm ; m (14H) 4,0 ppm
s (1H) 10,0 ; 10,3 ; 12,5 ppm

Exemple 17

Dichlorhydrate de (phénoxy-3 propyl)-1 (chloro-4 benzimidoyl)-4 pipérazine. F = 245-246 °C.

R.M.N. (DMSO $d_6$)
s (1H) 12,0 ppm ; s (4H) 7,5 ppm
s (1H) 10,1 ppm ; m (5H) 6,8 ppm
s (1H) 9,8 ppm ;

Exemple 18

Dichlorhydrate de (phénoxy-2 éthyl)-1 benzimidoyl-4 pipérazine. F = 250 °C.

R.M.N. (DMSO $d_6$)
s (5H) 7,7 ppm ; m (5H) 7,1 ppm
t (2H) 4,0 ppm ; m (10H) 3,5 ppm
s (1H) 9,8 ppm ; s (1H) 10,3 ppm
s (1H) 11,5 ppm

Exemple 19

Dichlorhydrate de (phénylthio-3 propyl)-1 benzimidoyl-4 pipérazine. F = 245 °C.

R.M.N. ($D_2O$)
s (5H) 7,8 ppm ; t (2H) 4,5 ppm
m (10H) 3,9 ppm ; s (5H) 8,1 ppm
t (2H) 3,4 ppm ; m (2H) 2,4 ppm

Exemple 20

Dichlorhydrate de (phénoxy-3 propyl)-1 benzimidoyl-4 pipérazine. F = 255 °C.

R.M.N. (DMSO $d_6$)
s (5H) 7,7 ppm ; t (2H) 4,0 ppm
m (10H) 3,5 ppm ; m (5H) 7,1 ppm
m (2H) 2,2 ppm ; s (1H) 9,9 ppm ; 10,3 ppm ; 11,5 ppm

Exemple 21

Dichlorhydrate de [(diméthyl-2,6 phénoxy)-2 éthyl]-1 (chloro-2 benzimidoyl)-4 pipérazine. F = 270 °C.

R.M.N. ($D_2O$)
s (6H) 2,4 ppm ; m (12H) 4,2 ppm
s (4H) 7,9 ppm ; s (3H) 7,3 ppm

Exemple 22

Dichlorhydrate de [(diméthyl-2,6 phénoxy)-2 éthyl]-1 (chloro-3 benzimidoyl)-4 pipérazine. F = 170 °C.

R.M.N. ($D_2O$)
s (8H) 2,6 ppm ; m (4H) 8,1 ppm
s (3H) 7,5 ppm ;

Exemple 23

Dichlorhydrate de [(diméthyl-2,6 phénoxy)-2 éthyl]-1 (chloro-2 méthyl-6 benzimidoyl)-4 pipérazine. F = 250 °C.

R.M.N. ($D_2O$)
s (6H) 2,4 ppm ; m (12H) 4,2 ppm
s (3H) 2,5 ppm ; s (3H) 7,2 ppm
s (3H) 7,8 ppm ;

Exemple 24

Dichlorhydrate de [(diméthyl-2,6 phénoxy)-2 éthyl]-1 (méthyl-4 benzimidoyl)-4 pipérazine. F = 270 °C.

R.M.N. ($D_2O$)
s (4H) 7,7 ppm ; s (6H) 2,4 ppm
s (3H) 7,15 ppm ; s (3H) 2,6 ppm
m (12H) 4,3 ppm ;

Exemple 25

Dichlorhydrate de [(diméthyl-2,6 phénoxy)-2 éthyl]-1 (méthyl-3 benzimidoyl)-4 pipérazine. F = 260 °C.

R.M.N. ($D_2O$)
s (4H) 7,7 ppm ; s (6H) 2,4 ppm
s (3H) 7,1 ppm ; s (3H) 2,5 ppm

Exemple 26

Dichlorhydrate de [(diméthyl-2,6 phénoxy)-2 éthyl]-1 (méthyl-2 benzimidoyl)-4 pipérazine. F = 264 °C.

R.M.N. ($D_2O$)
s (4H) 7,7 ppm ; s (3H) 7,3 ppm
s (6H) 2,4 ppm ; s (3H) 2,6 ppm

Exemple 27

Dichlorhydrate de [(diméthyl-2,6 phénoxy)-2 éthyl]-1 (benzyloxy-4 benzimidoyl)-4 pipérazine. F = 260 °C.

Exemple 28

Dichlorhydrate de (phényl-2 propyl)-1 (benzyloxy-4 benzimidoyl)-4 pipérazine. F = 280 °C.

Exemple 29

Dichlorhydrate de [(chloro-2 méthoxy-5 phénoxy)-2 éthyl]-1 benzimidoyl-4 pipérazine. F = 263 °C.

Exemple 30

Dichlorhydrate de [(diméthyl-3,5 chloro-4 phénoxy)-2 éthyl]-1 benzimidoyl-4 pipérazine. F = 250 °C.

Exemple 31

Dichlorhydrate de [(diméthyl-3,5 chloro-4 phénoxy)-3 propyl]-1 benzimidoyl-4 pipérazine. F = 265 °C

Exemple 32

Dichlorhydrate de [(diméthyl-2,6 phénoxy)-2 éthyl]-1 (hydroxy-4 benzimidoyl)-4 pipérazine. F = 235 °C

Exemple 33

Dichlorhydrate de (phényl-2 propyl)-1 (hydroxy-4 benzimidoyl)-4 pipérazine. F = 210 °C

Exemple 34

Dichlorhydrate de (phényl-2 propyl)-1 (benzyloxy-2 benzimidoyl)-4 pipérazine. F = 254 °C

Exemple 35

Dichlorhydrate de [(diméthyl-2,6 phénoxy)-2 éthyl]-1 (benzyloxy-3 benzimidoyl)-4 pipérazine. F = 245 °C

Exemple 36

Dichlorhydrate de [(diméthyl-2,6 phénoxy)-2 éthyl]-1 (benzyloxy-2 benzimidoyl)-4 pipérazine. F = 210 °C

Exemple 37

Dichlorhydrate de (phényl-2 propyl)-1 (benzyloxy-3 benzimidoyl)-4 pipérazine. F = 240 °C

Exemple 38

Dichlorhydrate de (phényl-2 propyl)-1 (hydroxy-3 benzimidoyl)-4 pipérazine. F = 270 °C

Exemple 39

Dichlorhydrate de [(diméthyl-2,6 phénoxy)-2 éthyl]-1 (hydroxy-2 benzimidoyl)-4 pipérazine. F = 200 °C

Exemple 40

Dichlorhydrate de [(diméthyl-2,6 phénoxy)-2 éthyl]-1 (hydroxy-3 benzimidoyl)-4 pipérazine. F = 200 °C

Les composés de formule I et leurs sels d'addition avec des acides pharmaceutiquement acceptables possèdent des actions intéressantes au niveau cardiovasculaire et plus particulièrement des propriétés antiarythmisantes et sont utilisables en thérapeutique pour la régulation du système cardiovasculaire.

L'activité correctrice des anomalies du rythme cardiaque a été mise en évidence chez le chien anesthésié au pentobarbital, présentant de l'arythmie et de la tachycardie centriculaire à la suite de l'injection d'une dose déterminée (40 à 100 $\gamma$/kg) d'ouabaïne.

Les composés de formule I, et plus particulièrement les composés des exemples 2, 6, 27, 28, 32, 35, 37, 38, 39, 40, administrés par voie veineuse à des doses uniques de 0,5 à 5 mg/kg, entraînant une protection vis-à-vis des arythmies provoquées par l'ouabaïne.

La toxicité par voie orale ($DL_{50}$) des composés de formule I chez le rat est comprise entre 400 et 1 100 mg/kg.

Les composés de formule I n'ont pas, par ailleurs, de propriétés secondaires gênantes.

La présente invention a donc également pour objet des compositions thérapeutiques contenant un composé de formule I ou l'un de ses sels d'addition avec des acides pharmaceutiquement acceptables, notamment en mélange avec un excipient pharmaceutiquement acceptable.

Les compositions thérapeutiques selon l'invention peuvent être administrées à l'homme par voie orale, ou par voie parentérale.

Ces compositions peuvent être notamment sous forme de gélules, comprimés, capsules ou ampoules injectables.

Ces compositions peuvent contenir suivant le mode d'administration notamment de 1 à 60 % en poids d'ingrédient actif en mélange avec un excipient pharmaceutiquement acceptable.

La dose journalière chez l'adulte peut être de 50 à 1 000 mg de principe actif par la voie orale et de 30 à 600 mg de principe actif par la voie parentérale.

On donnera ci-après des exemples de compositions thérapeutiques.

a) Ampoule injectable

| | |
|---|---|
| Composé de l'exemple 2 | 30 mg |
| Excipient tamponné q.s.p | 5 ml |

b) Gélule

| | |
|---|---|
| Composé de l'exemple 2 | 40 mg |
| Lactose | 153 mg |
| Talc | 5 mg |
| Stéarate de magnésium | 2 mg |

## Revendications

1. Composés de formule

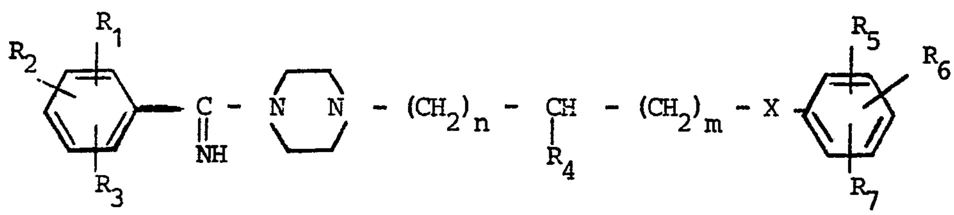

dans laquelle :

— les substituants $R_1$, $R_2$ et $R_3$ représentent indépendamment l'un de l'autre un atome d'hydrogène, un atome d'halogène, un radical trifluorométhyle, un radical alcoyle en $C_1$ à $C_6$ linéaire ou ramifié, un radical benzyloxy, ou un radical hydroxy.

— les substituants $R_5$, $R_6$ et $R_7$ représentent indépendamment l'un de l'autre un atome d'hydrogène, un atome d'halogène, ou un radical alcoyle en $C_1$ à $C_6$ linéaire ou ramifié.

— le substituant $R_4$ représente un atome d'hydrogène ou un radical alcoyle en $C_1$ à $C_6$.

— *m* est égal à 0 ou 1 et égal à 1 ou 2.

— X représente un atome d'oxygène, de soufre ou une simple liaison, ainsi que leurs sels d'addition avec des acides pharmaceutiques acceptables.

2. Composés de formule I telle que définie à la revendication 1, caractérisés en ce que

— les substituants $R_1$, $R_2$ et $R_3$ représentent indépendamment l'un de l'autre un atome d'hydrogène, un atome d'halogène, un radical trifluorométhyle, un radical alcoyle en $C_1$ à $C_4$, un radical benzyloxy ou un radical hydroxy,

— les substituants $R_5$, $R_6$ et $R_7$ représentent indépendamment l'un de l'autre un atome d'hydrogène, un atome d'halogène ou un radical alcoyle en $C_1$ à $C_4$,

— le substituant $R_4$ représente un atome d'hydrogène ou un radical méthyle.

— *m* est égal à 0 ou 1 et n est égal à 1 ou 2.

— X représente un atome d'oxygène, de soufre ou une simple liaison, ainsi que leurs sels d'addition avec des acides pharmaceutiques acceptables.

3. La [(diméthyl-2,6 phénoxy)-2 éthyl]-1 (benzimidoyl)-4 pipérazine et ses sels d'addition avec des acides pharmaceutiquement acceptables.

4. Composition thérapeutique ayant notamment des propriétés antiarythmisantes, caractérisée en ce qu'elle contient à titre de principe actif un composé selon l'une quelconque des revendications 1 à 3.

5. Procédé de préparation de composés de formule I, telle que définie à la revendication 1, caractérisé en ce que l'on fait réagir sur une pipérazine de formule

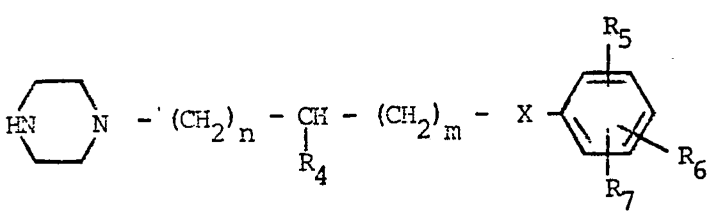

(III)

dans laquelle $R_4$, $R_5$, $R_6$ et $R_7$, *m* et *n*, ont la signification donnée pour la formule I, un composé choisi parmi

a) un iminoate d'alcoyle de formule

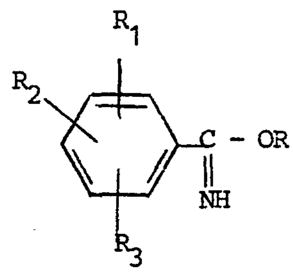

(II)

dans laquelle R est un radical alcoyle inférieur et $R_1$, $R_2$, $R_3$ ont les significations données pour la formule I.

b) une benzamidine de formule

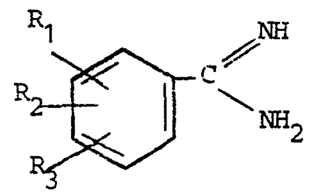

dans laquelle $R_1$, $R_2$, $R_3$ ont les significations données pour la formule I.

c) un iodhydrate de thiobenzimidate de S-méthyle de formule

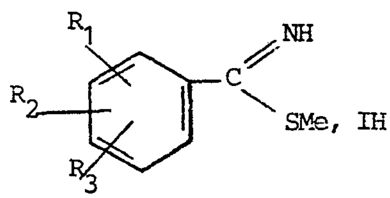

dans laquelle $R_1$, $R_2$, $R_3$ ont les significations données pour la formule I.

6. Procédé de préparation de composés de formule I telle que défini à la revendication 1, caractérisé en ce que l'on fait réagir un composé de formule

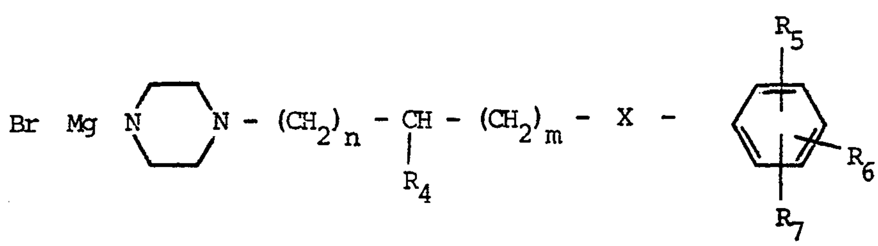

(VIII)

dans laquelle $R_4$, $R_5$, $R_6$, $R_7$, *m* et *n* ont les significations données à la revendication 1, sur un nitrile de formule

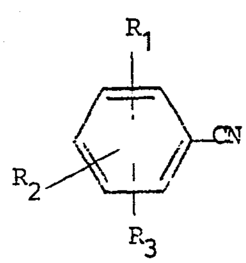

(IV)

dans laquelle $R_1$, $R_2$, $R_3$ ont les significations données à la revendication 1.

**Claims**

1. Compounds having the formula :

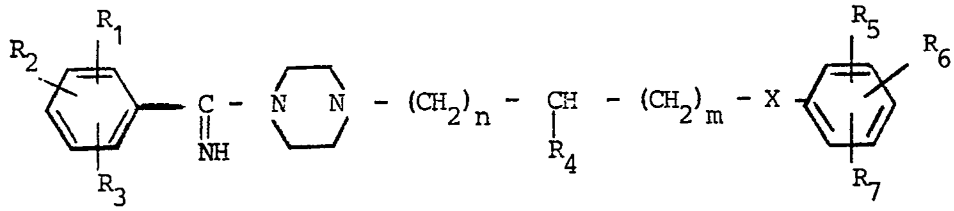

(I)

in which :

— the substituents $R_1$, $R_2$ and $R_3$ represent independently from each other a hydrogen atom, a halogen atom, a trifluoromethyl radical, a straight- or branched-chain $C_{1-6}$ alkyl radical, a benzyloxy radical or a hydroxy radical ;

— the substituents $R_5$, $R_6$ and $R_7$ represent independently from each other a hydrogen atom, a halogen atom, or a straight- or branched-chain $C_{1-6}$ alkyl radical ;

— the substituent $R_4$ represents a hydrogen atom or a $C_{1-6}$ alkyl radical ;

— *m* is equal to 0 or 1 and *n* is equal to 1 or 2 ;

— X represents an oxygen atom, a sulfur atom or a single bond, and their pharmaceutically acceptable acid addition salts.

2. Compounds of the formula I as defined in claim 1, wherein :

— the substituents $R_1$, $R_2$ and $R_3$ represent independently a hydrogen atom, a trifluoromethyl radical, a $C_{1-4}$ alkyl radical, a halogen atom, a benzyloxy radical or a hydroxy radical,

— the substituents $R_5$, $R_6$ and $R_7$ represent independently a hydrogen atom, a halogen atom or a $C_{1-4}$ alkyl radical,

— the substituent $R_4$ represents a hydrogen atom or a methyl radical,

— *m* is equal to 0 or 1 and *n* is equal to 1 or 2,

— X represents an oxygen atom, a sulfur atom, or a single bond, and their pharmaceutically acceptable acid addition salts.

3. 1-[2-(2,6-Dimethyl-phenoxy)-ethyl]-4-(benzimidoyl)-piperazine and its pharmaceutically acceptable acid addition salts.

4. Therapeutic composition having especially antiarrhytmic properties, characterised in containing a compound as claimed in any one of claims 1-3 as an active ingredient.

5. Process for the preparation of compounds of the formula (I) as claimed in claim 1, comprising reacting a piperazine having the formula :

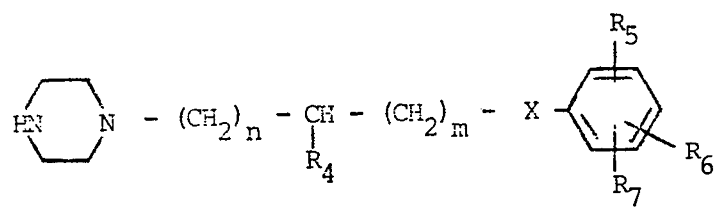

(III)

in which $R_4$, $R_5$, $R_6$, $R_7$, *m* and *n* have the meanings given for the formula (I) with a compound selected from :

a) a lower alkyl iminoate having the formula :

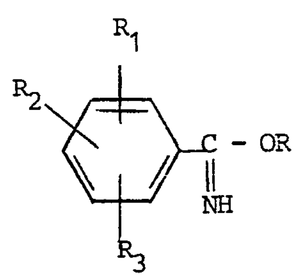

(II)

in which R is a lower alkyl radical and $R_1$, $R_2$ and $R_3$ have the meanings given for the formula (I) ;

b) a benzamidine having the formula :

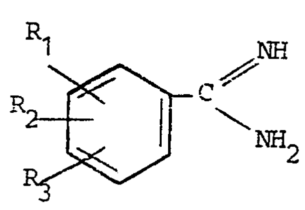

in which $R_1$, $R_2$ and $R_3$ have the meanings given for the formula (I) and

c) a S-methyl thiobenzimidate hydroiodide of the formula :

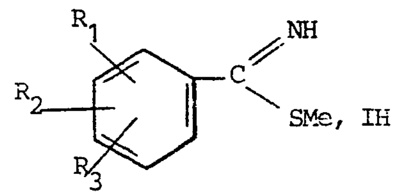

in which $R_1$, $R_2$ and $R_3$ have the meanings given for the formula (I).

6. Process for the preparation of compounds of the formula (I) as defined in claim 1, comprising reacting a compound having the formula :

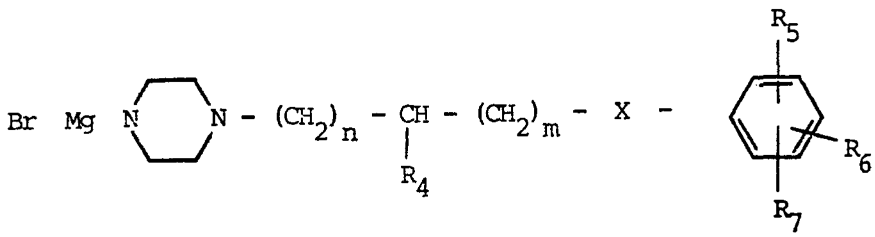

(VIII)

in which $R_4$, $R_5$, $R_6$, $R_7$ *m* and *n* have the meanings given in claim 1, with a nitrile having the formula :

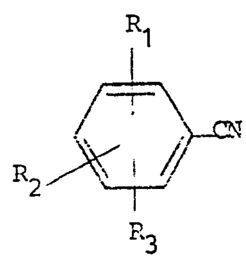

(IV)

in which $R_1$, $R_2$ and $R_3$ have the meanings given in claim 1.

**Ansprüche**

1. Verbindungen der Formel

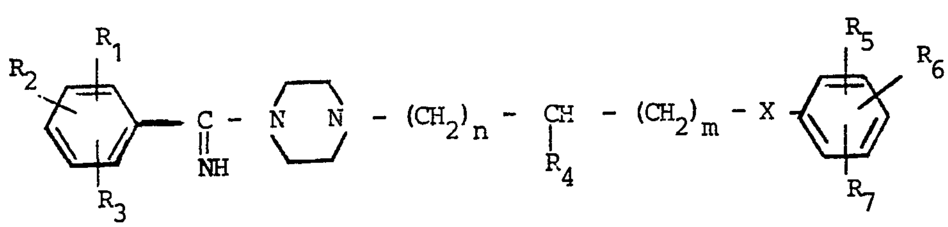

in der :

— die Substituenten $R_1$, $R_2$ und $R_3$ unabhängig voneinander ein Wasserstoffatom, ein Halogenatom, einen Trifluormethylrest, einen $C_1$-$C_6$-Alkylrest, einen Benzyloxyrest oder einen Hydroxyrest darstellen,

— die Substituenten $R_5$, $R_6$ und $R_7$ unabhängig voneinander ein Wasserstoffatom, ein Halogenatom oder einen linearen oder verzweigten $C_1$-$C_6$-Alkylrest darstellen,

— der Substituent $R_4$ ein Wasserstoffatom oder einen $C_1$-$C_6$-Alkylrest darstellt,

— *m* für 0 oder 1 und *n* für 1 oder 2 steht,

— X ein Sauerstoffatom, Schwefelatom oder eine Einfachbindung darstellt,

sowie ihre Additionssalze mit pharmazeutisch unbedenklichen Säuren.

2. Verbindungen der Formel I, wie sie in Anspruch 1 definiert wurde, dadurch gekennzeichnet, daß :

— die Substituenten $R_1$, $R_2$ und $R_3$ unabhängig voneinander ein Wasserstoffatom, ein Halogenatom, einen Trifluoromethylrest, einen $C_1$-$C_4$-Alkylrest, einen Benzyloxyrest oder einen Hydroxyrest darstellen,

— die Substituenten $R_5$, $R_6$ und $R_7$ unabhängig voneinander ein Wasserstoffatom, ein Halogenatom

oder einen $C_1$-$C_4$-Alkylrest darstellen,

— der Substituent $R_4$ ein Wasserstoffatom oder einen Methylrest darstellt,

— *m* für 0 oder 1 und *n* für 1 oder 2 steht,

— X ein Sauerstoffatom, ein Schwefelatom oder eine Einfachbindung darstellt,

sowie ihre Additionssalze mit pharmazeutisch unbedenklichen Säuren.

3. 1-[2-(2,6-Dimethylphenoxy)ethyl]4-(benzimidoyl)-piperazin und seine Additionssalze mit pharmazeutisch unbedenklichen Säuren.

4. Arzneimittelzubereitung mit insbesondere antiarhythmischen Eigenschaften, dadurch gekennzeichnet, daß sie als Wirkstoff eine Verbindung nach einem beliebigen der Ansprüche 1 bis 3 enthält.

5. Verfahren zur Herstellung von Verbindungen der Formel I, wie sie in Anspruch 1 definiert wurde, dadurch gekennzeichnet, daß man mit einem Piperazin der Formel

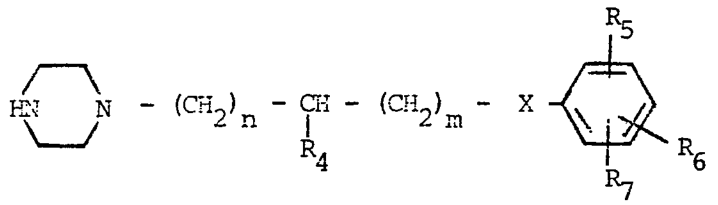

(III)

in der $R_4$, $R_5$, $R_6$ und $R_7$, *m* und *n* die für die Formel I angegebene Bedeutung haben, eine Verbindung umsetzt, die aus :

a) einem Alkyliminoat der Formel

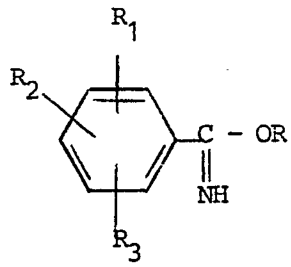

(II)

in der R ein niederer Alkylrest ist und $R_1$, $R_2$, $R_3$ die für die Formel I angegebenen Bedeutungen haben,

b) einem Benzamidin der Formel

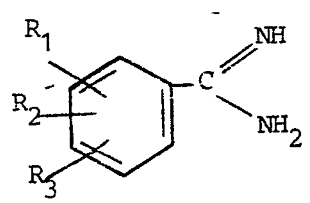

in der $R_1$, $R_2$, $R_3$ die für die Formel I angegebenen Bedeutungen haben,

c) einem Hydroiodid von S-Methylthiobenzimidat der Formel

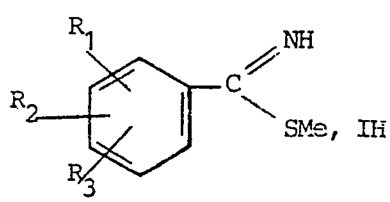

in der $R_1$, $R_2$, $R_3$ die für die Formel I angegebenen Bedeutungen haben, ausgewählt ist.

6. Verfahren zur Herstellung von Verbindungen der Formel I, wie sie in Anspruch 1 definiert wurde, dadurch gekennzeichnet, daß man eine Verbindung der Formel

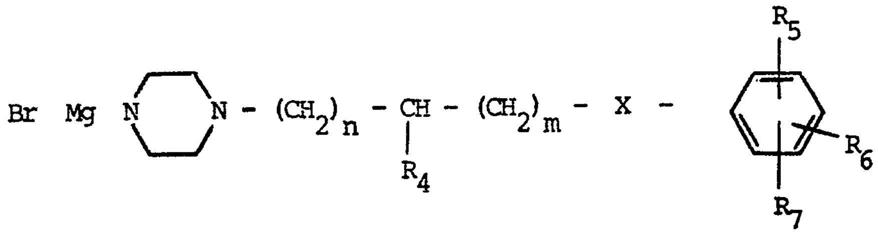
(VIII)

in der $R_4$, $R_5$, $R_6$, $R_7$, *m* und *n* die in Anspruch 1 angegebenen Bedeutungen haben, mit einem Nitril der Formel

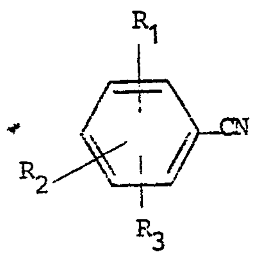
(IV)

in der $R_1$, $R_2$, $R_3$ die in Anspruch 1 angegebenen Bedeutungen haben, umsetzt.